## Europäisches Patentamt

## European Patent Office

## Office européen des brevets

(11) Veröffentlichungsnummer: **0 377 822**
**A1**

(12) **EUROPÄISCHE PATENTANMELDUNG**

(21) Anmeldenummer: **89122202.8**

(22) Anmeldetag: **01.12.89**

(51) Int. Cl.5: **C12P 7/18, C12P 7/58**

(30) Priorität: **10.12.88 DE 3841702**

(43) Veröffentlichungstag der Anmeldung:
**18.07.90 Patentblatt 90/29**

(84) Benannte Vertragsstaaten:
**BE CH DE GB LI NL**

(71) Anmelder: **Forschungszentrum Jülich GmbH**
**Postfach 1913**
**D-5170 Jülich(DE)**

(72) Erfinder: **Bringer-Meyer, Stephanie**
**Buchheimer Strasse 23**
**D-4150 Krefeld(DE)**
Erfinder: **Sahm, Hermann, Prof. Dr.**
**Wendelinusstrasse 71**
**D-5170 Jülich(DE)**

(54) **Verfahren zur fermentativen Gewinnung von Sorbit und Gluconsäure und dafür geeignetes Zellmaterial.**

(57) Für die Gewinnung von Sorbit und Gluconsäure bzw. Gluconat durch Fermentation in wäßrigen Glucose/Fructosemischungen werden durch Einfriertechnik permeabilisierte Zellen von Zymomonas mobilis verwendet, die vorzugsweise durch Einfrieren von in verdünnter Pufferlösung von pH 6 his 7 resuspendierten Zellzentrifugaten bei etwa -20° C und Auftauen bei Zimmertemperatur erhalten worden sind. Vorzugsweise werden für die Fermentation Zellkonzentrationen von 20 his 60 g/l Zelltrockenmasse permeabilisierter Zellen verwendet. Durch die Verwendung der durch Gefrieren und Auftauen permeabilisierten Zellen wird eine erheblich raschere Umsetzung von Glucose/Fructose in Sorbit und Gluconsäure bzw. Gluconat erreicht.

EP 0 377 822 A1

## Verfahren zur fermentativen Gewinnung von Sorbit und Gluconsäure und dafür geeignetes Zellmaterial

Die Erfindung bezieht sich auf ein Verfahren zur Gewinnung von Sorbit und Gluconsäure bzw. Gluconat ausgehend von wäßrigen Glucose/Fructosemischungen durch Fermentation in Gegenwart von permeabilisierten Zellen von Zymomonas mobilis, und sie umfaßt dafür geeignetes permeabilisiertes Zellmaterial.

Es ist bekannt, daß in wäßrigen Glucose/Fructosemischungen enzymatisch mit Hilfe von Glucose-Dehydrogenase und Sorbit-Dehydrogenase bei gleichzeitiger Anwesenheit von Cofaktoren Sorbit und Gluconsäure bzw. Gluconat entstehen. Dieses Verfahren setzt die fortlaufende Zugabe oder Regenerierung der Cofaktoren voraus.

Es wurde daher bereits ein Verfahren zur mikrobiellen Umwandlung von Glucose und Fructose in wäßriger Lösung mit Hilfe von Glucose/Fructose-Transhydrogenase enthaltenden Bakterien und insbesondere von Zymomonas mobilis entwickelt (US-PS 4 755 467), bei dem entsalzte zellfreie Extrakte oder immobilisierte Zellen oder mit phosphatfreiem Puffer gewaschene, nichtwachsende Zellen verwendet werden, die ggf. permeabel gemacht sind, wofür im konkreten Beispiel eine Toluol-Behandlung diente. Alternativ sollen Gluconatkinase-negative Mutanten gebildet und angewandt werden.

Es wurde nun überraschenderweise gefunden, daß die Umsetzung von Glucose/Fructose in Sorbit und Gluconsäure bzw. Gluconat erheblich rascher abläuft, wenn man durch Gefrieren und Auftauen permeabilisierte Zellen einsetzt.

Das erfindungsgemäße Verfahren ist demgemäß dadurch gekennzeichnet, daß man durch Einfriertechnik permeabilisierte Zellen verwendet.

Zwar ist die Einfriertechnik als Mittel zur Permeabilisierung von Zellen (z.B. E. Coli) unter einer Vielzahl von anderen Techniken bekannt (siehe Hansruedi Felix. Anal. Biochem. 120 (1982) 211 - 234; insb. 221), jedoch war keinesfalls zu erwarten, daß gerade mittels der Einfriertechnik im vorliegenden Falle eine sehr erhebliche Verbesserung erzielt werden könnte.

Vorzugsweise werden die durch Einfrieren und Auftauen zu permeabilisierenden Zellen nach ihrer Abtrennung vom Kulturmedium in verdünnter Pufferlösung von pH 6 - 7 suspendiert und in dieser Form auf etwa -20°C eingefroren und anschließend bei Zimmertemperatur wieder aufgetaut. Als Puffer dient insbesondere Natriumcitratpuffer von pH 6,5, in dem die Zellmasse insbesondere in Mengen von 50 - 150 g/l Zelltrockenmasse suspendiert wird. Zweckmäßigerweise kann die Zellmasse vor dem Einfrieren ein-oder mehrfach mit Citratpuffer gewaschen werden.

Die Einfriertemperatur sollte in Bereichen liegen, wo die Keimbildungszahl der Kristallisation von Wasser schon erheblich ist. Dabei ist zu berücksichtigen, daß die Kühlraumtemperatur schwerlich momentan die gesamte Zellmasse erfaßt, deren mittlere Temperatur sich nur mehr oder minder schnell der Kühltemperatur annähert. Als obere Grenze der Einfriertemperatur ist je nach Geornetrie der Zellmasse etwa -10°C anzusehen. Gute Ergebnisse wurden bei einem Einfrieren von Suspensions röhrchen (Durchmesser 2,5 cm, Länge 4,5 cm, Wandmaterial Polyethylen) in einem bei ca. -20°C befindlichen Gefrierschrank erhalten. Auch niedrigere Temperaturwerte sind anwendbar, und es kann beispielsweise bis unter -30°C und tiefer gekühlt werden.

Das Auftauen kann besonders einfach bei Raumtemperatur durch Entnahme der Proben aus dem Gefrierschrank erfolgen oder aber, indem man diese in ein entsprechend temperiertes Wasserbad bringt und so den Auftauvorgang etwas beschleunigt, wodurch eine gewisse Verbesserung der Nützlichkeit der permeabilisierten Zellen für die Sorbit- und Gluconsäuregewinnung erreicht wird.

Solche permeabilisierten Zellmaterialien können etwa analog zu Enzympräparaten erzeugt, bevorratet und vertrieben werden.

Für die Sorbit- und Gluconsäuregewinnung werden insbesondere wäßrige Glucose/Fructosemischungen mit jeweiliger Konzentration von 100 - 270 g/l, vorzugsweise je 200 g/l Hexose verwendet. Die Zellkonzentration liegt insbesondere im Bereich von 20 - 50 g/l Zelltrockenmasse, vorzugsweise bei ca. 40 g/l Zelltrockenmasse. Der pH-Wert kann im Bereich von 5,5 -7,0 gewählt werden und liegt vorzugsweise bei 6,5, wobei die pH-Kontrolle vorzugsweise durch Titration mit 2 M $Na_2CO_3$ erfolgt.

Vorzugsweise wird in gepufferter Lösung gearbeitet, ins besondere in Natriumcitratpuffer (0,1 M; pH 6,5) oder mit anderen, jedoch phosphatfreien Pufferlösungen, die im genannten pH-Bereich puffern.

Die Temperatur liegt zweckmäßigerweise zwischen 30°C und 42°C und vorzugsweise bei 39°C.

Nachfolgend wird die Erfindung anhand von Beispielen erläutert.

Beispiel 1:

Gewinnung von permeabilisierten Zellen.

Zellanzucht:

pH-statisch im Fermenter Substrat: 150 g/1 Saccharose Medium (g/100 ml $H_2O$): Hefeextrakt: 0,5; $KH_2PO_4$: 0,1; $(NH_4)_2SO_4$: 0,1; $MgSO_4$ x 7 $H_2O$: 0,05;
pH 5,0
Wachstumszeit: ca. 16 Stunden

Zellernte:

bei einer optischen Dichte ($OD_{550\,nm}$) von 13,4
Abzentrifugieren bei 10000 x g, 20 Minuten;
Resuspendieren der Zellen in 0,1 M Natriumcitratpuffer, pH 6,5
Abzentrifugieren und Aufnahme der Zellen in Citratpuffer (ca. 120 g/l Zelltrockenmasse).

Einfrieren der Suspension bei -20° C.

Auftauen der Zellen bei Raumtemperatur.
Abzentrifugieren der Zellen bei 48000 x g, 20 Minuten.
Resuspendieren der Zellen in 0,1 M Natriumcitratpuffer, pH 6,5.
Abzentrifugieren bei 48000 x g, 10 Minuten.
Resuspendieren in Natriumcitratpuffer, pk 6,5, Zellkonzentration ca. 30 g/l Zelltrockenmasse.

Beispiel 2:

Gewinnung von Sorbit- und Gluconsäure mit Hilfe der permeabilisierten Zellen:

Glucose: 234 g/l (1,3 M)
Fructose: 234 g/l (1,3 M)
Zellkonzentration: 43 g/l Zelltrockenmasse pH-Wert: 6,5
Temperatur: 39° C
Puffer: Natriumcitrat, 0,1 M, pH 6,5
Titration: mit 2 M $Na_2CO_3$
Zeit für die Umsetzung: 5 Stunden
Sorbit: 233 g/l (1,28 M)
Gluconsäure: 247 g/l (1,26 M)
Fructose: 3,6 g/l (0,02 M)
Glucose: 7,2 g/l (0,04 M)

**Ansprüche**

1. Verfahren zur Gewinnung von Sorbit und Gluconsäure bzw. Gluconat ausgehend von wäßrigen Glucose/Fructrosemischungen durch Fermentation in Gegenwart von permeabilisierten Zellen von Zymomonas mobilis,
**dadurch gekennzeichnet,**
daß man durch Einfriertechnik permeabilisierte Zellen verwendet.

2. Verfahren nach Anspruch 1,
**dadurch gekennzeichnet,**
daß man permeabilisierte Zellen verwendet, die durch Einfrieren von in verdünnter Pufferlösung von pH 6 bis 7 resuspendierten Zellzentrifugaten bei etwa -20° C und Auftauen bei Zimmertemperatur erhalten worden sind.

3. Verfahren nach Anspruch 2,
**dadurch gekennzeichnet,**
daß man permeabilisierte Zellen verwendet, die durch Einfrieren von in 0,1 M Natriumcitrat-Puffer von pH 6,5 resuspendierten Zellzentrifugaten mit 50 bis 150 g/l Zelltrockenmasse und Auftauen erhalten worden sind.

4. Verfahren nach Anspruch 3,
**dadurch gekennzeichnet,**
daß man permeabilisierte Zellen verwendet, die durch Einfrieren und Auftauen wiederholt resuspendierter Zellen erhalten worden sind.

5. Verfahren nach Anspruch 4,
**dadurch gekennzeicnnet,**
daß man permeabilisierte Zellen verwendet, die nach dem Auftauen ein- oder mehrfach in Citratpuffer resuspendiert worden sind.

6. Verfahren nach einem der vorangehenden Ansprüche,
**dadurch gekennzeichnet,**
daß man die Fermentation unter Einsatz von Zellkonzentrationen permeabilisierter Zellen von 20 bis 60 g/l (Zelltrockenmasse) durchführt.

7. Permeabilisiertes Zellmaterial von Zymomonas mobilis aus durch Einfriertechnik permeabilisierten Zellen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | EP-A-0 322 723 (FUJISAWA PHARM. CO.) <br> * Insgesamt * <br> --- | 1,2,4,6,7 | C 12 P 7/18 <br> C 12 P 7/58 |
| A | APPLIED MICROBIOLOGY AND BIOTECHNOLOGY, Band 29, 1988, Seiten 19-24; U.H. CHUN et al.: "The simultaneous production of sorbitol from fructose and gluconic acid from glucose using an oxidoreductase of Zymomonas mobilis" <br> * Insgesamt * <br> --- | 1,2,4,6,7 | |
| A ‹ | EP-A-0 212 517 (KERNFORSCHUNGSANLAGE JÜLICH) <br> ----- | | |

| | | | RECHERCHIERTE SACHGEBIETE (Int. Cl.5) |
|---|---|---|---|
| | | | C 12 P |

Der vorliegende Recherchenbericht wurde für alle Patentansprüche erstellt

| Recherchenort | Abschlußdatum der Recherche | Prüfer |
|---|---|---|
| DEN HAAG | 16-03-1990 | DESCAMPS J.A. |